# FASCICULE DE BREVET EUROPEEN

(11) **EP 1 884 261 B1**
(45) Date de publication et mention de la délivrance du brevet: **13.10.2010**
(21) Numéro de dépôt: 07113247.6
(22) Date de dépôt: 26.07.2007
(51) Int. Cl.: A61Q 5/06, A61K 8/60, A61K 8/73

(54) **Composition cosmétique comprenant au moins un polysaccharide de type carraghénane lambda en association avec au moins un agent tensioactif non-ionique de type alkylpolyglucoside, procédé de traitement cosmétique des fibres kératiniques et utilisation de la composition**
Kosmetische Zusammensetzung enthaltend mindestens ein Polysaccharid vom Lambda-Carrageenan Typ in Verbindung mit mindestens ein nichtionischen Tensid vom Alkylpolyglukosid Typ, kosmetische Behandlungsmethode für Keratinfasern und Verwendung der Zusammensetzung
Cosmetic composition containing at least one lambda carrageenan type polysaccharide in association with at least one non-ionic alkyl polyglucoside type surface active agent, cosmetic treatment method of keratin fibres and use of the composition

(30) Priorité: 04.08.2006 FR 0607158
(43) Date de publication de la demande: 06.02.2008
(73) Titulaire: L'Oréal, 75008 Paris (FR)
(72) Inventeur: Restle, Serge M., 95390, Saint-Prix (FR); Laurent, Ludivine, 92400, Courbevoie (FR)
(74) Mandataire: Dossmann, Gérard

(56) Documents cités:
- WO-A-2006/064151
- US-A- 5 670 471
- US-A1- 2006 134 049
- US-B1- 6 296 859

## Description

La présente invention concerne une composition cosmétique pour le traitement des fibres kératiniques, en particulier des fibres kératiniques humaines telles que les cheveux, comprenant au moins un polysaccharide de type carraghénane lambda et au moins un agent tensio-actif particulier, un procédé de traitement cosmétique la mettant en oeuvre ainsi qu'une utilisation de cette composition pour la fixation ou le soin desdites fibres.

Dans le domaine du coiffage, en particulier parmi les produits capillaires destinés à la mise en forme et/ou au maintien de la coiffure, les compositions capillaires sont généralement constituées d'une solution le plus souvent alcoolique ou aqueuse et d'un ou de plusieurs polymères fixants en mélange avec divers adjuvants cosmétiques.

Ces compositions peuvent se présenter sous forme de gels ou de mousses capillaires qui sont généralement appliquées sur des cheveux mouillés avant d'effectuer un brushing ou un séchage.

En particulier, les gels capillaires sont notamment constitués d'un ou de plusieurs polymères épaississants ou agents gélifiants en association avec un ou plusieurs polymères fixants qui ont le plus souvent pour fonction de former un film à la surface des fibres kératiniques à fixer.

Récemment, les carraghénanes ont été utilisés en tant que polymères fixants dans des gels de coiffage ou dans des aérosols. En effet, il est connu du document EP-A-1 199 064 de mettre en oeuvre un carraghénane ou un mélange de ceux-ci et un additif particulier pour obtenir un gel solide et stable pour le traitement capillaire.

Les carraghénanes sont des polysaccharides qui constituent les parois cellulaires de diverses algues rouges (Rhodophycées) appartenant aux familles des Gigartinaceae, Hypneaceae, Furcellariaceae et Polyideaceae. Ils comportent des longues chaînes galactanes, polyélectrolytes anioniques. Leur masse moléculaire peut être supérieure à 10⁶ u.m.a. (unité de masse atomique). Ces polymères linéaires, formés par des motifs disaccharides, sont composés par deux unités D-galactopyranoses liées alternativement par des liaisons α- et β-. Ce sont des polysaccharides très sulfatés (20-50 %) et les résidus α-D-galactopyranosyles peuvent être sous forme 3',6'-anhydro.

Initialement, les carraghénanes ont été subdivisés en deux familles suivant leur solubilité dans le chlorure de potassium (KCl). Les fractions solubles dans le KCl ont été désignées par les préfixes "Kappa", tandis que les termes "Lambda" ont été réservés à celles insolubles. Plus tard, les classifications ont été basées sur le nombre, la position de groupements sulfates ainsi que la présence de pont 3',6'-anhydro sur les résidus β-D-galactopyranosyles. Ceci a abouti aux quatre grandes familles : κ, λ, β, ω.

Les différents types de carraghénanes n'existent pas à l'état pur, mais sous forme d'hybrides. Ainsi à l'état naturel, les κ et τ-carraghénanes se présentent sous une forme hybride Kappa-iota mais l'une des deux structures peut prédominer sur l'autre. L'état hybride κ-τ-d'une structure peut être élucidé en utilisant des enzymes spécifiques, qui permettent d'enrichir ou de diminuer la teneur en l'une des deux formes. Les carraghénanes peuvent coexister avec leurs précurseurs. Les carraghénanes de différentes familles d'appartenance peuvent coexister dans une structure hybride. Ex.: carraghénane de Euchema gelatinae : une hybride de β-carraghénane, composant majeur, et de κ, γ-carraghénanes.

Il est connu du document EP-A-0 445 659, de combiner les carraghénanes avec un agent tensio-actif non-ionique oxyéthyléné particulier, dans le but d'obtenir une force d'adhésion particulière entre les cheveux, de produire un film doux et élastique, de produire un effet de douceur des cheveux secs et de maintenir la mise en forme des cheveux. Toutefois, les compositions décrites ne permettent pas d'obtenir un effet traitant suffisant sur cheveux humides avant brushing.

De même, il est connu du document JP-A-05-178718 de combiner une poudre de carraghénane dont les particules ont une taille définie avec un additif cosmétique, tel qu'un tensioactif dans le but d'obtenir après gonflement de ladite poudre une composition ayant un toucher doux et un effet non collant. Ce document divulgue préférentiellement l'utilisation de la carraghénane kappa.

D'autre part, une composition contenant la λ-carraghénane sans agent moussant ne permet pas d'obtenir une mousse coiffante de texture satisfaisante.

Il existe donc un réel besoin de trouver des compositions cosmétiques, notamment pour le coiffage, qui permettent de remédier aux inconvénients cités ci-dessus.

De manière surprenante et avantageuse, la Demanderesse vient de découvrir qu'en associant au moins une λ-carraghénane avec des alkylpolyglucosides, il était possible d'obtenir une composition capillaire avec de bonnes propriétés de soin à l'application sur cheveux humides, tout en conservant d'excellentes propriétés coiffantes et cosmétiques sur cheveux séchés. De plus, l'utilisation de cette λ-carraghénane au sein d'une telle association permet d'obtenir des gels de meilleures textures, moins cassants, moins durs et plus faciles à appliquer.

D'autre part, sous forme aérosol, cette association permet d'obtenir une mousse coiffante de texture satisfaisante.

La présente invention a donc notamment pour objet une composition cosmétique pour le traitement des fibres kératiniques, en particulier des fibres kératiniques humaines telles que les cheveux, comprenant, dans un milieu cosmétiquement acceptable :
- au moins un polysaccharide de type carraghénane lambda, et
- au moins un agent tensioactif non-ionique de type alkylpolyglucoside.

En particulier, elle porte sur une composition cosmétique pour le traitement des fibres kératiniques, en particulier des fibres kératiniques humaines telles que les cheveux, comprenant, dans un milieu cosmétiquement acceptable,
- au moins un polysaccharide de type carraghénane lambda, et
- au moins un agent tensioactif non-ionique de type alkylpolyglucoside,
la teneur en polysaccharide de type carraghénane lambda étant comprise entre 0,1 et 30 % en poids du poids total de la composition, les valeurs aux bornes étant exclues.

L'invention a également pour objet un procédé de traitement cosmétique mettant en oeuvre la composition cosmétique selon l'invention.

Un autre objet de la présente invention consiste en une utilisation de la composition cosmétique selon l'invention pour la fixation des cheveux ou pour le soin des cheveux.

D'autres objets, caractéristiques, aspects et avantages de l'invention apparaîtront encore plus clairement à la lecture de la description et des exemples qui suivent.

Par le mot « coiffage », on entend le fait de fixer et/ou de maintenir la forme de la coiffure.

La composition selon l'invention comprend au moins un polysaccharide de type carraghénane lambda.

De préférence, le polysaccharide de type carraghénane lambda utilisé selon la présente invention n'est pas modifié chimiquement.

De préférence, le poids moléculaire (PM) du polysaccharide est compris entre 100 000 et 1 000 000 u.m.a. Encore plus préférentiellement, le poids moléculaire est compris entre 250 000 et 800 000 u.m.a.

Par le mot "compris", on entend le fait que les valeurs aux bornes sont exclues de la gamme de valeurs décrite.

A titre de polysaccharide de type carraghénane lambda utilisable dans le cadre de la présente invention, on peut citer le SATIAGUM UTC 10 de la société DEGUSSA et le WELGEENAN ED 1039 de la société EUROGUM.

Le polysaccharide de type carraghénane lambda peut être présent dans la composition en une quantité variant de 0,2 à 20 % en poids, et plus préférentiellement de 0,5 à 15 % en poids, par rapport au poids total de la composition cosmétique.

Les agents tensioactifs non-ioniques de type alkylpolyglucoside alkyl utilisables dans la composition selon la présente invention sont des produits bien connus en soi, qui peuvent être plus particulièrement représentés par la formule générale (I) suivante :

R₁-O-(R₂-O)ₐ-(L)_{b} (I)

dans laquelle :
R₁ désigne un radical alkyle et/ou alcényle linéaire ou ramifié comportant environ de 8 à 24 atomes de carbone, un radical alkylphényle dont le groupe alkyle linéaire ou ramifié comporte environ de 8 à 24 atomes carbone,
R₂ désigne un radical alkylène comportant de 2 à 4 atomes de carbone,
L désigne un sucre réduit comportant de 5 à 6 atomes de carbone,
a désigne une valeur allant de 0 à 10, et
b désigne une valeur allant de 1 à 15.
Les alkylpolyglucosides préférés selon la présente invention sont des composés de formule (I) dans laquelle :
R₁ désigne plus particulièrement un radical alkyle et/ou alcényle linéaire ou ramifié comportant de 9 à 14 atomes de carbone,
a désigne une valeur allant de 0 à 3 et plus particulièrement encore égale à zéro,
L désigne le glucose, le fructose ou le galactose. Plus particulièrement, L désigne le glucose.

Le degré de polymérisation (S) du saccharide, i.e. la valeur de b dans la formule (I), peut aller de 1 à 15. Selon l'invention, on préfère les sucres réduits contenant 80 %, ou plus, de sucres dont le degré de polymérisation (S) prend une valeur allant de 1 à 4.
Des composés de formule (I) sont notamment représentés par les produits vendus par la société HENKEL sous la dénomination APG, tels que les produits APG 300, APG 350, APG 500, APG 550, APG 625, APG base 10-12; les produits vendus par la société SEPPIC sous les dénominations TRITON CG 110 (ou ORAMIX CG 110) et TRITON CG 312 (ou ORAMIX NS 10) ; ceux vendus par la société B.A.S.F. sous la dénomination LUTENSOL GD 70 ; vendus par la société HENKEL sous les dénominations PLANTAREN 1200, PLANTAREN 1300, PLANTAREN 2000, et PLANTACARE 2000, PLANTACARE 818, PLANTACARE 1200.

Le (ou les) alkylpolyglucoside(s) peut (ou peuvent) être présent(s) dans la composition à une teneur comprise entre 0,05 et 30 % en poids, de préférence entre 0,1 et 15 % en poids, du poids total de la composition cosmétique.

Les compositions cosmétiques conformes à l'invention peuvent se présenter sous forme de crème, de mousse (aérosol ou non), de pâte ou de gel. Plus préférentiellement, ce gel présente une viscosité supérieure à 200 cPs à 25 °C à un taux de cisaillement de 1 s⁻¹. Tout particulièrement, cette viscosité est comprise entre 500 et 500 000 cPs à 25 °C, de préférence entre 500 et 100 000 cPs à 25 °C, et encore plus préférentiellement entre 500 et 50 000 cPs à 25 °C à un taux de cisaillement de 1 s⁻¹. Elle peut être mesurée à l'aide d'un viscosimètre de type cône-plan.

La composition selon l'invention peut également peut-être lavante ou non, et peut comprendre une base lavante connue de l'homme de l'art.

La composition peut en outre comprendre des composants optionnels.

La composition cosmétique selon l'invention peut comprendre en outre au moins une silicone.

Dans tout ce qui suit, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

De préférence, la silicone est une silicone oxyalkylénée.

Par silicone oxyalkylénée, on entend toute silicone comportant au moins un groupement oxyalkyléné de type (-CₓH₂ₓO-)ₐ dans lequel x peut varier de 2 à 6, et a est supérieur ou égal à 2.

Les silicones oxyalkylénées utilisables dans la composition cosmétique sont choisies parmi les formules générales (VI), (VII), (VIII) ou (IX) suivantes: 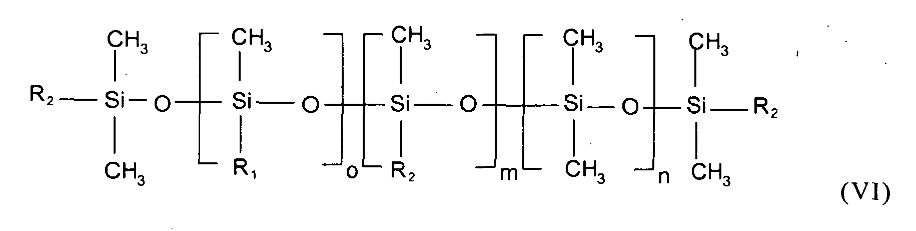 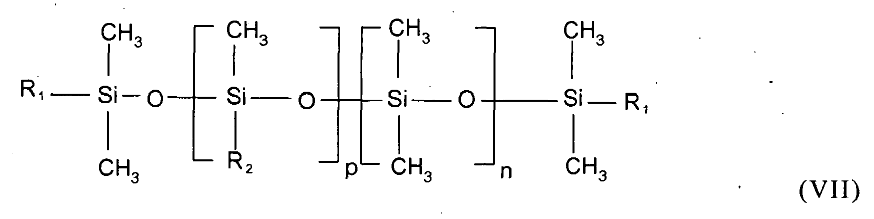 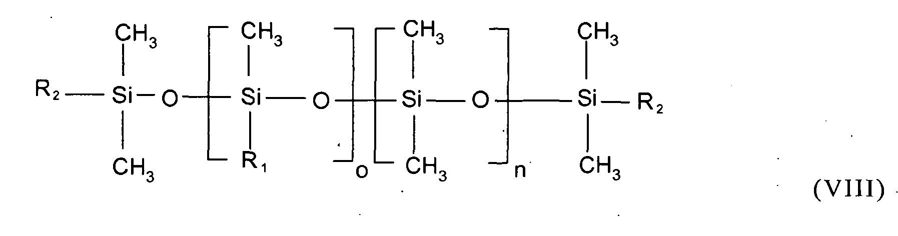 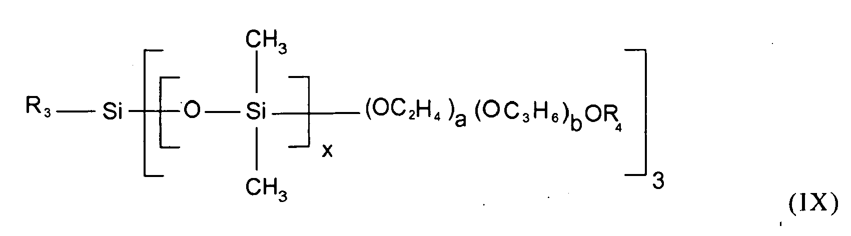 dans lesquelles :
R₁, identiques ou différents, représentent un radical alkyle, linéaire ou ramifié, en C₁-C₃₀ ou phényle,
R₂, identiques ou différents, représentent un radical -C_{c}H_{2c}-O-(C₂H₄O)ₐ(C₃H₆O)_{b}-R₅ ou un radical -C_{c}H_{2c}-O-(C₄H₈O)ₐ-R₅,
R₃ et R₄, identiques ou différents, désignent un radical alkyle, linéaire ou ramifié, en C₁ à C₁₂, et de préférence le radical méthyle,
R₅, identiques ou différents, sont choisis parmi un atome d'hydrogène, un radical alkyle, linéaire ou ramifié, comprenant de 1 à 12 atomes de carbone, un radical alcoxy, linéaire ou ramifié, comprenant de 1 à 6 atomes de carbone, un radical acyle, linéaire ou ramifié, comprenant de 2 à 30 atomes de carbone, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement éventuellement substitué par un ou deux radicaux aminoalkyle substitués, -CO(CH₂)_{d}COOM, -COCHR₇(CH₂)_{d}COOM, -NHCO(CH₂)_{d}OH, -NH₃Y, un groupement phosphate,
M, identiques ou différents, désignent un atome d'hydrogène, Na, K, Li, NH₄ ou une amine organique,
R₇ désigne un atome d'hydrogène ou un radical SO₃M,
d varie de 1 à 10,
m varie de 0 à 20,
n varie de 0 à 500,
o varie de 0 à 20,
p varie de 1 à 50,
a varie de 0 à 50,
b varie de 0 à 50,
a + b est supérieur ou égal à 2,
c varie de 0 à 4,
x varie de 1 à 100,
Y⁻ représente un anion minéral ou organique monovalent tel que halogénure (chlorure, bromure), sulfate, carboxylate (acétate, lactate, citrate),
sous réserve que lorsque la silicone est de formule (VII) avec R₅ désignant hydrogène alors n est supérieur à 12.

De telles silicones sont par exemple commercialisées par la société GOLDSCHMIDT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DC 3225 C, Q2-5220, Q25354, Q2-5200, par la société RHODIA CHIMIE sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WSL, SILWAX DCA 100, SILTECH AMINE 65, par la société FANNING CORPORATION sous les dénominations FANCORSIL SLA, FANCORSIL LIM1, par la société PHOENIX sous la dénomination PECOSIL.

Ces silicones sont notamment décrites dans les brevets US-A-5,070,171, US-A-5,149,765, US-A-5,093,452 et US-A-5,091,493.

De préférence, on utilise les silicones polyoxyalkylénées répondant aux formules générales (VII) ou (VIII). Plus particulièrement, ces formules répondent à au moins une des conditions suivantes, et de préférence toutes les conditions suivantes :
c est égal à 2 ou 3,
R₁ désigne le radical méthyle,
R₅ représente un radical méthyle, un radical acyle en C₁₂-C₂₂, -CO(CH₂)_{d}COOM, d et M étant tels que définis précédemment,
a varie de 2 à 25 et plus particulièrement de 2 à 15,
b est égal à 0,
n varie de 0 à 100,
p varie de 1 à 20.

Les silicones polyoxyalkylénées peuvent également être choisies parmi les silicones comprenant au moins un motif de formule (X) suivante :

([Z(R₂SiO)_{q}R'₂SiZO][(CₙH₂ₙO)ᵣ])ₛ (X)

dans laquelle:
- R et R', identiques ou différents, représentent un radical hydrocarboné monovalent en C₁-C₃₀,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.
- s est un nombre supérieur ou égal à 4, de préférence compris entre 4 et 1 000 et encore plus particulièrement entre 5 et 300.
- Z, identiques ou différents, représentent un groupe organique divalent qui est lié à l'atome de silicium adjacent par une liaison carbone-silicium et/ou au bloc polyoxyalkylène (CₙH₂ₙO) par un atome d'oxygène,
- le poids moléculaire moyen de chaque bloc siloxane est compris entre environ 400 et environ 10 000 u.m.a., celui de chaque bloc polyoxyalkylène étant compris entre environ 300 et environ 10 000 u.m.a.,
- les blocs siloxane représentent de 10 % environ à 95 % environ en poids du copolymère bloc,
- le poids moléculaire moyen en nombre du copolymère bloc pouvant aller de 2 500 à 1 000 000 et de préférence compris entre 3 000 et 200 000 et encore plus particulièrement entre 6 000 et 100 000.

R et R' sont préférentiellement choisis parmi le groupe comprenant les radicaux alkyles linéaires ou ramifiés comme par exemple les radicaux méthyle, éthyle, propyle, butyle, pentyle, hexyle, octyle, décyle, dodécyle, les radicaux aryles comme par exemple phényle, naphtyle, les radicaux aralkyles ou alkylaryles comme par exemple benzyle, phényléthyle, les radicaux tolyle, xylyle.

Z, identiques ou différents, sont de préférence choisis parmi les groupes suivants : -R"-, -R"-CO-, -R"-NHCO-, -R"-NH-CO-NH-R"'-, - R"-OCONH-R"'-NHCO- ; R" étant un groupe alkylène divalent, linéaire ou ramifié en C₁-C₆, comme par exemple l'éthylène, le propylène ou le butylène, linéaire ou ramifié et R"' étant un groupe alkylène divalent ou un groupe arylène divalent comme -C₆H₄-, -C₆H₄-C₆H₄-, -C₆H₄-CH₂-C₆H₄-, -C₆H₄-C(CH₃)₂C₆H₄-.

Encore plus préférentiellement, Z, identiques ou différents, représentent un radical alkylène divalent, plus particulièrement un radical -C₃H₆- ou un radical C₄H₈, linéaire ou ramifié.

La préparation des copolymères blocs est décrite dans la demande européenne EP-A-0 492 657.

De tels produits sont par exemple commercialisés sous la dénomination SILICONE FLUID FZ-2172 par la société OSI.

Les silicones utilisées peuvent se présenter sous forme de solutions aqueuses, c'est-à-dire solubilisée ou éventuellement sous forme de dispersions ou micro-dispersion, ou d'émulsions aqueuses.

La (ou les) silicone(s) utilisable(s) dans la composition cosmétique peut (ou peuvent) aussi être une (ou des) gomme(s) de silicone.

Les gommes de silicone utilisables dans la composition cosmétique sont notamment des polydiorganosiloxanes ayant des poids moléculaires moyens en poids élevés compris entre 200 000 et 1 000 000 u.m.a. utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxane/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SE 30 correspondant à une diméthicone, ayant un poids moléculaire de 500 000 u.m.a., solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % en poids de gomme SE 30 et 85 % en poids d'une huile SF 96.

La ou les silicones utilisables dans la composition cosmétique peuvent aussi être des silicones aminées.

Par silicone aminé, on entend toute silicone comportant au moins une fonction amine primaire, secondaire, tertiaire ou un groupement ammonium quaternaire.

Les silicones aminées utilisées dans la composition cosmétique selon la présente invention sont choisies parmi :

(a) les composés répondant à la formule (XI) suivante :

(R¹)ₐ(T)₍₃₋ₐ₎-Si[OSi(T)₂]ₙ-[OSi(T)_{b}(R¹)_{(2-b)}]ₘ-OSi(T)₍₃₋ₐ₎-(R¹)ₐ (XI)

dans laquelle :
- T, identiques ou différents, représentent un atome d'hydrogène, ou un radical phényle, hydroxyle (-OH), alkyle en C₁-C₈, de préférence méthyle, ou alcoxy en C₁-C₈, de préférence méthoxy,
- a désigne le nombre 0 ou un nombre entier de 1 à 3, et de préférence 0,
- b désigne 0 ou 1, et en particulier 1,
- m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
- R¹ est un radical monovalent de formule -C_{q}H_{2q}L dans laquelle q est un nombre entier allant de 2 à 8 et L est un groupement aminé, éventuellement quaternisé, choisi parmi les groupements :
   -N(R²)-CH₂-CH₂-N(R²)₂ ;
   -N(R²)₂ ;
   -N⁺(R²)₃ Q⁻ ;
   -N⁺(R²)(H)₂ Q⁻ ;
   -N⁺(R²)₂HQ⁻ ;
   -N(R²)-CH₂-CH₂-N⁺(R²)(H)₂ Q⁻ ;
   dans lesquels R² peut désigner un atome d'hydrogène, un phényle, un benzyle, ou un radical hydrocarboné saturé monovalent, par exemple un radical alkyle en C₁-C₂₀, et Q⁻ représente un ion halogénure tel que par exemple fluorure, chlorure, bromure ou iodure.

En particulier, les silicones aminées correspondant à la définition de la formule (XI) sont choisies parmi les composés correspondant à la formule (XII) suivante : 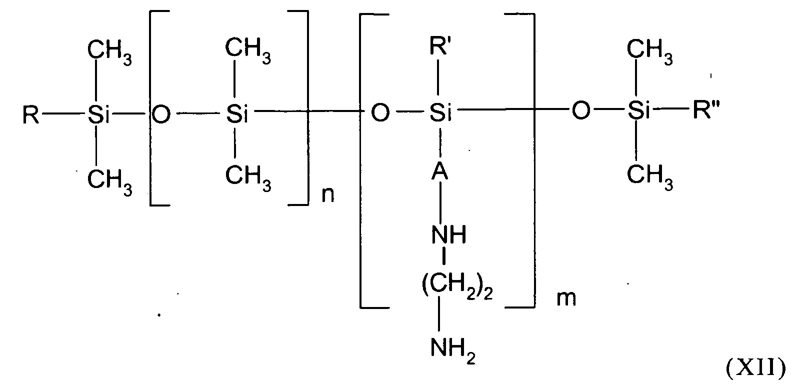 dans laquelle :
R, R' et R", identiques ou différents, désignent un radical alkyle en C₁-C₄, de préférence CH₃ ; un radical alcoxy en C₁-C₄, de préférence méthoxy ; ou OH ;
A représente un radical alkylène, linéaire ou ramifié, en C₃-C₈, de préférence en C₃-C₆ ;
m et n sont des nombres entiers dépendant du poids moléculaire et dont la somme est comprise entre 1 et 2000.

Selon une première possibilité, R, R' et R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 u.m.a. environ. Les composés de ce type sont dénommés dans le dictionnaire CTFA, "amodiméthicone".

Selon une deuxième possibilité, R, R' et R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy/alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶ u.m.a. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R et R", différents, représentent un radical alcoxy en C₁-C₄ ou hydroxyle, l'un au moins des radicaux R ou R" est un radical alcoxy, R' représente un radical méthyle et A représente un radical alkylène en C₃. Le rapport molaire hydroxy/alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200 000 u.m.a. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Selon une quatrième possibilité, R et R" représentent un radical hydroxyle, R' représente un radical méthyle et A est un radical alkylène, en C₄-C₈, de préférence en C₄. Par ailleurs, m et n sont tels que la masse moléculaire moyenne en poids du composé est comprise entre 2000 et 10⁶ u.m.a. Plus particulièrement, n est compris entre 0 et 1999 et m est compris entre 1 et 2000, la somme de n et m étant comprise entre 1 et 2000.

Un produit de ce type est notamment commercialisé sous la dénomination DC 28299 par Dow Corning.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (XI) est en particulier le polymère dénommé dans le dictionnaire CTFA "triméthylsilylamodiméthicone", répondant à la formule (XIII) suivante: 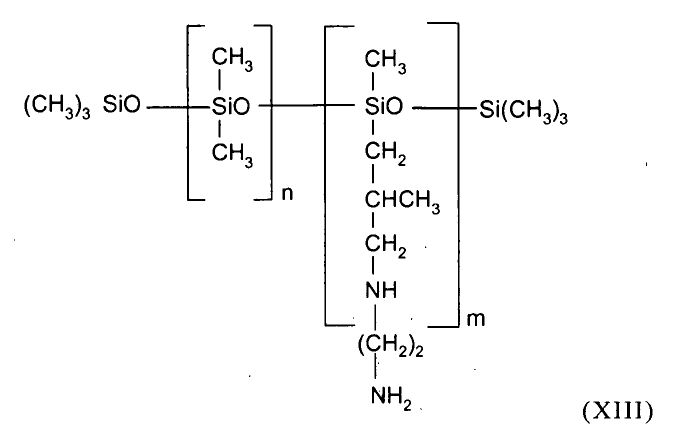 dans laquelle n et m ont les significations données ci-dessus conformément à la formule (XI).

De tels composés sont décrits par exemple dans EP-A-0 095 238 ; un composé de formule (XIII) est par exemple vendu sous la dénomination Q2-8220 par la société OSI.

(b) les composés répondant à la formule (XIV) suivante : 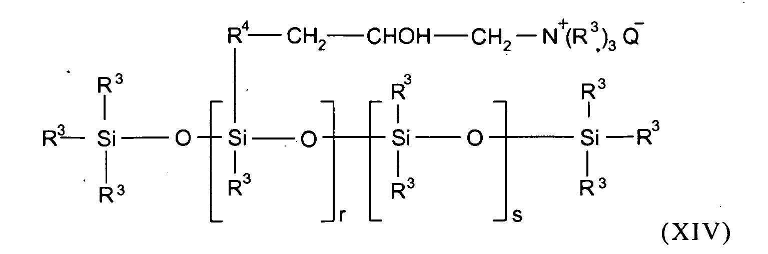 dans laquelle :
R³, identiques ou différents, représentent un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels composés sont décrits plus particulièrement dans le brevet US-A-4,185,087.

Un composé entrant dans cette classe est celui vendu par la Société Union Carbide sous la dénomination "Ucar Silicone ALE 56".

c) les silicones ammonium quaternaire de formule (XV) : 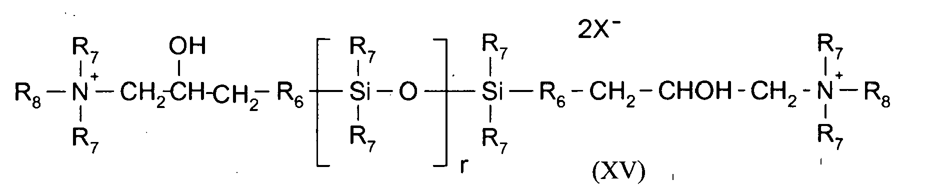 dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈, relié au Si par une liaison Si-C ;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
X⁻ est un anion tel qu'un ion halogénure, notamment chlorure ou un sel d'acide organique (acétate ...) ;
r représente une valeur statistique moyenne allant de 2 à 200 et en particulier de 5 à 100 ;

Ces silicones sont par exemple décrites dans la demande EP-A-0 530 974.

d) les silicones aminées de formule (XVI) : 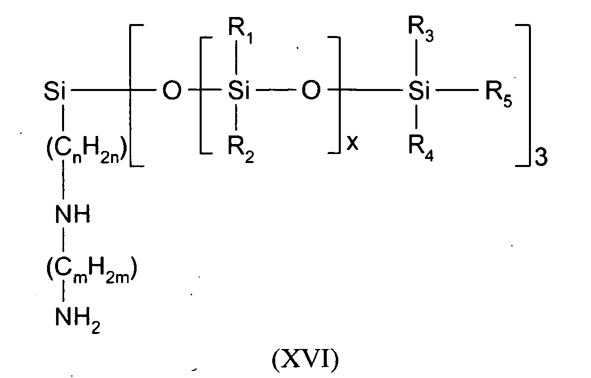 dans laquelle :
R₁, R₂, R₃ et R₄, identiques ou différents, désignent un radical alkyle en C₁-C₄ ou un groupement phényle,
R₅ désigne un radical alkyle en C₁-C₄ ou un groupement hydroxyle,
n est un entier variant de 1 à 5,
m est un entier variant de 1 à 5,
et dans laquelle x est choisi de manière telle que l'indice d'amine soit compris entre 0,01 et 1 meq/g.

Les silicones particulièrement préférées sont les polysiloxanes à groupements aminés tels que les amodiméthicones ou les triméthylsilylamodiméthicones (CTFA 4^{ème} édition 1997), et encore plus particulièrement les silicones à groupements ammonium quaternaire.

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule : 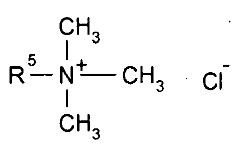 dans laquelle :
R⁵ désigne des radicaux alcényle et/ou alcoyle en C₁₄-C₂₂ dérivés des acides gras du suif, et connu sous la dénomination CTFA "tallowtrimonium chloride", en association avec un agent de surface non ionique de formule :

   C₉H₁₉-C₆H₄-(OC₂H₄)₁₀-OH,

   connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent dé surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (XVI) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule : C₁₂H₂₅-(OCH₂-CH₂)₆-OH, connu sous la dénomination CTFA "isolaureth-6", et du propylèneglycol.

La (ou les) silicone(s) est (ou sont) présente(s) dans la composition dans une quantité variant de 0,1 à 30 % en poids, de préférence en une quantité variant de 0,2 à 20 % en poids, et encore plus préférentiellement dans une quantité variant de 0,5 à 10 % en poids, par rapport au poids total de la composition.

La composition cosmétique selon l'invention peut comprendre en outre un corps gras non siliconé tels que les huiles végétales, animales, minérales et synthétiques, les alcools gras, les acides gras et les cires.

Le (ou les) corps gras est (ou sont) présent(s) dans la composition dans une quantité variant de 0,1 à 30 % en poids, de préférence en une quantité variant de 0,2 à 20 % en poids, et encore plus préférentiellement dans une quantité variant de 0,5 à 10 % en poids, par rapport au poids total de la composition.

Par « alcool gras », on entend au sens de la présente invention, tout alcool gras pur saturé ou insaturé, linéaire ou ramifié, comportant au moins 8 atomes de carbone. L'alcool gras peut être oxyalkyléné ou glycérolé.

L'alcool gras peut présenter la structure R-OH, dans laquelle R désigne un radical saturé ou insaturé, linéaire ou ramifié, comportant de 8 à 40 atomes de carbone et de préférence de 8 à 30 ; R désigne de préférence un groupement alkyle en C₁₂-C₂₄ ou alkényle en C₁₂-C₂₄. R peut être substitué par un ou plusieurs groupements hydroxy.

A titre d'exemple d'alcools gras, on peut citer les alcools laurique, cétylique, dodécylique, décylique, stéarylique, oléïque, béhénique, linoléique, undécylénique, palmitoléïque, arachidonique, érucique et leurs mélanges.

L'alcool gras peut représenter un mélange d'alcools gras, ce qui signifie que dans un produit commercial peuvent coexister plusieurs espèces d'alcools gras, sous forme d'un mélange.

A titre de mélange d'alcools gras, on peut citer l'alcool cétylstéarylique ou cétéarylique.

Avantageusement, l'alcool gras non oxyalkyléné est solide ou pâteux à la température de 25°C. Par « alcool gras solide ou pâteux à 25°C », on entend au sens de la présente invention un alcool gras présentant une viscosité mesurée avec un rhéomètre avec un taux de cisaillement de 1s⁻¹ supérieure ou égale à 1 Pa.s.

De préférence, les alcools gras utilisés dans la composition cosmétique selon l'invention sont l'alcool cétylique et l'alcool cétéarylique.

Par acides gras, on entend au sens de la présente invention, tout acide carboxylique pur saturé ou insaturé, linéaire ou ramifié comportant au moins 8 atomes de carbone. A titre d'exemples d'acide gras, on peut citer l'acide laurique, l'acide oléique.

La composition cosmétique peut comprendre en outre un ou plusieurs polymères fixants additionnels.

Par « polymère fixant », on entend au sens de la présente invention tout polymère permettant de conférer une forme ou de maintenir une forme ou une coiffure donnée.

La composition cosmétique peut comprendre en outre un ou plusieurs polymères fixants additionnels différents des polymères de l'invention.

Les polymères fixants utilisables dans la composition cosmétique selon l'invention sont notamment choisis parmi les polymères cationiques, anioniques, amphotères ou non ioniques et leurs mélanges autres que le matériau fixant décrits ci-dessus.

Par « polymère cationique », on entend au sens de la présente invention, tout polymère comprenant des groupements cationiques et/ou des groupements ionisables en groupements cationiques.

Les polymères fixants cationiques utilisables dans la composition cosmétique selon l'invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :

(1) les homopolymères ou copolymères d'esters ou d'amides acryliques ou méthacryliques, à fonctions aminées, comportant au moins un des motifs de formules suivantes : 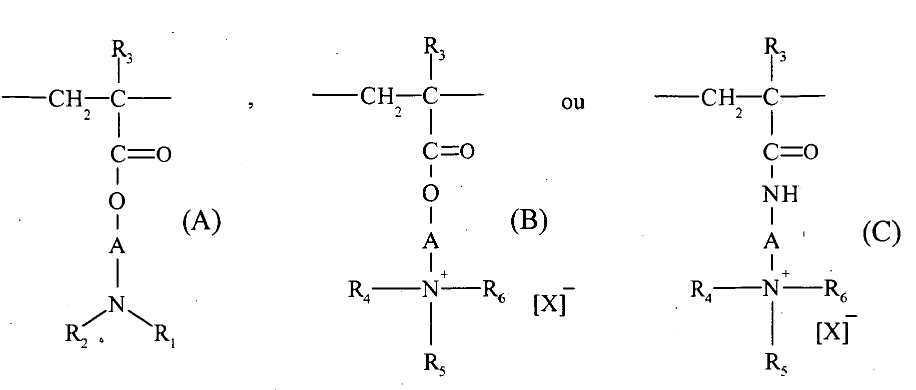 dans lesquelles :
R₁ et R₂, identiques ou différents, représentent chacun un atome d'hydrogène ou un groupe alkyle ayant de 1 à 6 atomes de carbone ;
R₃ désigne un atome d'hydrogène ou un groupe CH₃ ;
A est un groupe alkyle linéaire ou ramifié, comportant de 1 à 6 atomes de carbone ou un groupe hydroxyalkyle comportant de 1 à 4 atomes de carbone ;
R₄, R₅, R₆, identiques ou différents, représentent un groupe alkyle ayant de 1 à 18 atomes de carbone ou un groupe benzyle ;
[X]⁻ désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, des méthacrylamides, des diacétone-acrylamides, des acrylamides et des méthacrylamides substitués sur l'azote par des groupes alkyle inférieur en C₁-C₄, des groupes dérivés des acides acryliques ou méthacryliques ou de leurs esters, des vinyllactames tels que la vinylpyrrolidone ou le vinylcaprolactame, et des esters vinyliques.

Ainsi, parmi ces copolymères de la famille (1), on peut citer :
- les copolymères d'acrylamide et de méthacrylate de diméthylaminoéthyle quaternisés au sulfate de diméthyle ou avec un halogénure de diméthyle, tels que celui vendu sous la dénomination HERCOFLOC^{®} par la société HERCULES,
- les copolymères d'acrylamide et de chlorure de méthacryloyloxyéthyltriméthylammonium décrits, par exemple, dans la demande de brevet EP-A-0 080 976 et vendus sous la dénomination BINAQUAT P 100 par la société CIBA GEIGY,
- les copolymères d'acrylamide et de méthosulfate de méthacryloyloxyéthyltriméthylammonium, tels que celui vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT^{®}" par la société ISP comme, par exemple, "GAFQUAT^{®} 734" ou "GAFQUAT^{®} 755", ou bien les produits dénommés "COPOLYMER^{®} 845, 958 et 937". Ces polymères sont décrits en détail dans les brevets français Nos 2 077 143 et 2 393 573,
- les terpolymères méthacrylate de diméthylaminoéthyle/vinylcaprolactame/vinylpyrrolidone tels que le produit commercialisé sous la dénomination GAFFIX^{®} VC 713 par la société ISP, et
- les copolymères vinylpyrrolidone/méthacrylamide de diméthylaminopropyle quaternisé tels que notamment le produit commercialisé sous la dénomination "GAFQUAT^{®} HS 100" par la société ISP.

(2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C15, JAGUAR C17 par la société MEYHALL.

(3) les copolymères quaternaires de vinylpyrrolidone et de vinylimidazole.

(4) les chitosanes ou leurs sels ; les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.

(5) les dérivés de cellulose cationiques tels que les copolymères de cellulose ou de dérivés de cellulose greffés avec un monomère hydrosoluble comportant un ammonium quaternaire, et décrits notamment dans le brevet US 4,131,576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyldiallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 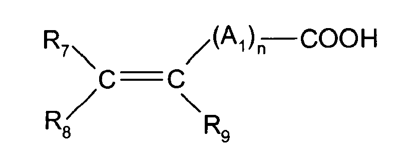 dans laquelle :
n est un nombre entier allant de 0 à 10,
A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre,
R₇ désigne un atome d'hydrogène, un groupement phényle ou benzyle,
R₈ désigne un atome d'hydrogène, un groupement alkyle inférieur ou carboxyle,
R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :

A) les homo- ou copolymères d'acide acrylique ou méthacrylique ou leurs sels et en particulier les produits vendus sous les dénominations VERSICOL^{®} E ou K par la société ALLIED COLLOID, et ULTRAHOLD^{®} par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.

B) les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371 ou proposés sous la dénomination QUADRAMER par la Société AMERICAN CYANAMID. On peut également citer les terpolymères acide méthacrylique/acrylate d'éthyle/acrylate de tertiobutyle tels que le produit commercialisé sous la dénomination LUVIMER^{®} 100 P par la société BASF.

On peut aussi citer les copolymères acide méthacrylique/acide acrylique/acrylate d'éthyle/méthacrylate de méthyle en dispersion aqueuse, commercialisé sous la dénomination AMERHOLD^{®} DR 25 par la société AMERCHOL.

C) les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.

D) les copolymères d'acides ou d'anhydrides carboxyliques monoinsaturés en C₄-C₈ choisis parmi :
- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2,047,398, 2,723,248, 2,102,113, le brevet GB n° 839 805. Des produits commerciaux sont notamment ceux vendus sous les dénominations GANTREZ^{®} AN ou ES par la société ISP.
- les copolymères comprenant (i) un ou plusieurs motifs anhydrides maléique, citraconique, itaconique et (ii) un ou plusieurs monomères choisis parmi les esters allyliques ou méthallyliques
comportant éventuellement un ou plusieurs groupements acrylamide, méthacrylamide, α-oléfine, esters acryliques ou méthacryliques, acides acrylique ou méthacrylique, ou vinylpyrrolidone dans leur chaîne,

les fonctions anhydrides de ces copolymères étant éventuellement monoestérifiées ou monoamidifiées.

Ces polymères sont par exemple décrits dans les brevets FR-A-2 350 384 et FR-A-2 357 241 de la demanderesse.

E) les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 u.m.a., ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan^{®} 500 et Flexan^{®} 130 par National Starch. Ces composés sont décrits dans le brevet FR-A-2 198 719.
- les sels d'acides polyacrylamide-sulfoniques tels que ceux mentionnés dans le brevet US-A-4,128,631, et plus particulièrement l'acide polyacrylamidoéthylpropane-sulfonique vendu sous la dénomination COSMEDIA POLYMER HSP 1180 par Henkel.

De préférence, les polymères fixants anioniques sont choisis parmi les copolymères d'acide acrylique tels que les terpolymères acide acrylique/acrylate d'éthyle/N-tertio-butylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX^{®} A par la société BASF.

Parmi les polymères fixants anioniques cités ci-dessus, on préfère plus particulièrement utiliser dans le cadre de la présente invention les copolymères méthylvinyléther/anhydride maléique monoestérifiés vendus sous la dénomination GANTREZ^{®} ES 425 par la société ISP, les terpolymères acide acrylique/acrylate d'éthyle/N-tertio-butylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :

(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, ou l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain US-A-3,836,537.

(2) les polymères comportant des motifs dérivant :
a) d'au moins un monomère choisi parmi les acrylamides ou les méthacrylamides substitués sur l'atome d'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertio-butylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant de 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA (4^{ème} Ed., 1991) est octylacrylamide/acrylates/butylaminoethyl methacrylate copolymer, tels que les produits vendus sous la dénomination AMPHOMER^{®} ou LOVOCRYL^{®} 47 par la société NATIONAL STARCH.

(3) les polyaminoamides réticulés et acylés partiellement ou totalement dérivant de polyaminoamides de formule générale (XVII) suivante :

-[CO-R₁₀-CO-Z]- (XVII)

dans laquelle :
R₁₀ représente un groupe divalent dérivé d'un acide dicarboxylique saturé, d'un acide aliphatique mono ou dicarboxylique à double liaison éthylénique, d'un ester d'un alcanol inférieur ayant de 1 à 6 atomes de carbone de ces acides ou d'un groupe dérivant de l'addition de l'un quelconque desdits acides avec une amine bis-primaire ou bis-secondaire, et
Z désigne un groupe dérivant d'une polyalkylène-polyamine bis-primaire, mono- ou bis-secondaire et de préférence contient les motifs suivants :
   a) dans les proportions de 60 à 100 % en moles, le groupe de formule générale (XVIII) : 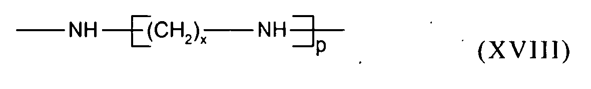 où x=2 et p=2 ou 3, ou bien x=3 et p=2 ;
      ce groupe dérivant de la diéthylène-triamine, de la triéthylène-tétraamine ou de la dipropylène-triamine ;
   b) dans les proportions de 0 à 40 % en moles, le groupe (XVIII) ci-dessus, dans lequel x=2 et p=1 et qui dérive de l'éthylène-diamine, ou le groupe dérivant de la pipérazine : 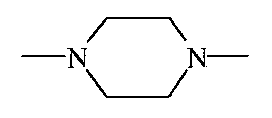
   c) dans les proportions de 0 à 20 % en moles, le groupe -NH-(CH₂)₆-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane sultone ou de leurs sels.

Les acides carboxyliques saturés sont choisis de préférence parmi les acides ayant de 6 à 10 atomes de carbone tels que les acides adipique, triméthyl-2,2,4-adipique, triméthyl-2,4,4-adipique, ou téréphtalique, et les acides à double liaison éthylénique comme, par exemple, les acides acrylique, méthacrylique, ou itaconique.

Les alcane-sultones utilisées dans l'acylation sont de préférence la propane- ou butane-sultone, les sels des agents d'acylation sont de préférence les sels de sodium ou de potassium.

(4) les polymères comportant des motifs zwittérioniques de formule : 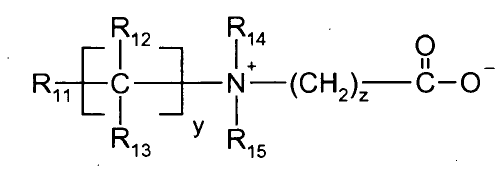 dans laquelle :
R₁₁ désigne un groupement insaturé polymérisable tel qu'un groupement acrylate, méthacrylate, acrylamide ou méthacrylamide,
y et z représentent un nombre entier allant de 1 à 3,
R₁₂ et R₁₃, identiques ou différents, représentent un atome d'hydrogène, un groupe méthyle, éthyle ou propyle,
R₁₄ et R₁₅, identiques ou différents, représentent un atome d'hydrogène ou un groupe alkyle de telle façon que la somme des atomes de carbone dans R₁₄ et R₁₅ ne dépasse pas 10.

Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.

A titre d'exemple, on peut citer les copolymères méthacrylate de méthyle/diméthylcarboxyméthylammonioéthylméthacrylate de méthyle, tels que le produit vendu sous la dénomination DIAFORMER Z301 par la société SANDOZ.

(5) les polymères dérivés du chitosane comportant des motifs monomères répondant aux formules suivantes, ainsi que les sels formés par ces composés avec des bases ou des acides : 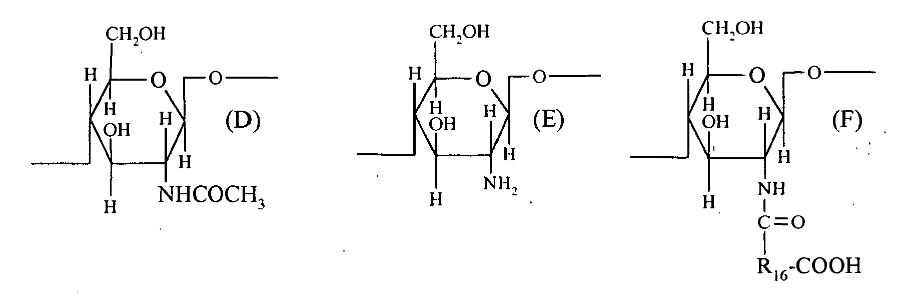 le motif (D) étant présent dans des proportions comprises entre 0 et 30 % en moles, le motif (E) dans des proportions comprises entre 5 et 50 % en moles et le motif (F) dans des proportions comprises entre 30 et 90 % en moles, étant entendu que dans ce motif (F), R₁₆ représente un groupe de formule : 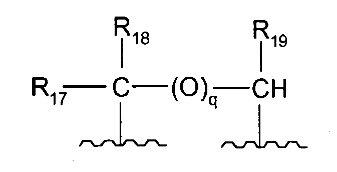 dans laquelle :
si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
ou si q=1, R₁₇, R₁₈ et R₁₉ représentent chacun un atome d'hydrogène.

(6) les polymères comportant des motifs répondant à la formule générale (XIX) sont, par exemple, décrits dans le brevet FR-A-1 400 366: 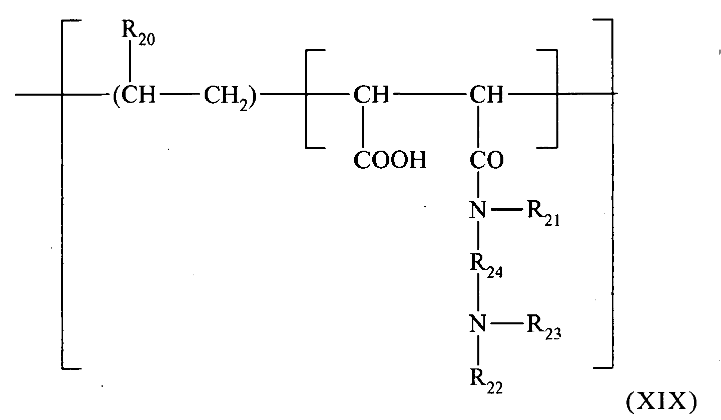 dans laquelle :
R₂₀ représente un atome d'hydrogène, un groupe CH₃O, CH₃CH₂O, ou phényle,
R₂₁ désigne un atome d'hydrogène ou un groupe alkyle inférieur tel que méthyle, ou éthyle,
R₂₂ désigne un atome d'hydrogène ou un groupe alkyle inférieur en C₁-C₆ tel que méthyle, ou éthyle,
R₂₃ désigne un groupe alkyle inférieur en C₁-C₆ tel que méthyle, ou éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, et R₂₂ ayant les significations mentionnées ci-dessus.

(7) les polymères dérivés de la N-carboxyalkylation du chitosane comme le N-(carboxyméthyl)chitosane ou le N-(carboxybutyl)chitosane, vendu sous la dénomination "EVALSAN" par la société JAN DEKKER.

(8) les polymères amphotères du type -D-X-D-X choisis parmi :
a) les polymères obtenus par action de l'acide chloracétique ou le chloracétate de sodium sur les composés comportant au moins un motif de formule (XX) :

   -D-X-D-X-D- (XX)

   où D désigne un groupe pipérazine : 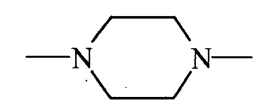 et X désigne le symbole E ou E' ; E ou E', identiques ou différents, désignant un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
b) les polymères de formule (XXI):

   -D-X-D-X- (XXI)

   où D désigne un groupe pipérazine : 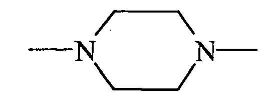 et X désigne le symbole E ou E' et au moins une fois E' ; E ayant la signification indiquée ci-dessus et E' étant un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) les copolymères alkyl(C₁-C₅)vinyléther/anhydride maléique modifiés partiellement par semi-amidification avec une N,N-dialkylaminoalkylamine telle que la N,N-diméthylaminopropylamine ou par semi-estérification avec un N,N-dialkylaminoalcanol. Ces copolymères peuvent également comporter d'autres comonomères vinyliques tels que le vinylcaprolactame.

Parmi les polymères fixants amphotères décrits ci-dessus, les plus particulièrement préférés selon l'invention sont ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthylcarboxyméthylammonioéthylméthacrylate de méthyle vendu par exemple sous la dénomination DIAFORMER^{®} Z301 1 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHODIA CHIMIE ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyle des polymères non ioniques mentionnés ci-dessus ont, de préférence, de 1 à 6 atomes de carbone.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP-A-0 751 162, EP-A-0 637 600, EP-A-0 648 485 et FR-A-2 743 297 dont la demanderesse est titulaire, ainsi que dans les demandes EP-A-0 656 021 et WO-A-94/03510 de la société BASF, et EP-A-0 619 111 de la société National Starch.

Comme polyuréthanes convenant particulièrement bien dans la présente invention, on peut citer les produits commercialisés sous les dénominations LUVISET P.U.R.^{®} et LUVISET^{®} Si P.U.R. par la société BASF.

Le (ou les) polymère(s) fixant(s) additionnel(s) est (ou sont) présent(s) dans la composition cosmétique selon l'invention en une quantité variant de 0,01 à 20 % en poids, de préférence de 0,05 à 15 % en poids, et encore plus préférentiellement de 0,1 à 10 % en poids, par rapport au poids total de la composition cosmétique.

La composition selon l'invention peut également contenir des tensioactifs additionnels.

Les tensioactifs ioniques utilisés dans la composition cosmétique peuvent également être des tensioactifs cationiques.

A titre d'exemple de tensioactifs cationiques utilisables dans la composition cosmétique, on peut notamment citer les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux qui présentent la formule générale (II) suivante : 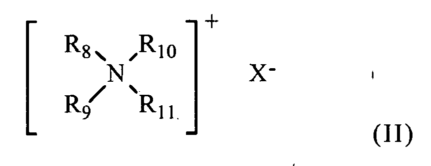 dans laquelle :
   les radicaux R₈ à R₁₁, identiques ou différents, représentent un radical aliphatique, linéaire ou ramifié, comportant de 1 à 30 atomes de carbone, ou un radical aromatique tel que aryle ou alkylaryle ;
   X⁻ est un anion choisi dans le groupe des halogénures, phosphates, acétates, lactates, alkyl(C₂-C₆)sulfates, alkyl- ou alkylaryl-sulfonates.

Dans la formule (II) ci-dessus, les radicaux aliphatiques peuvent comporter des hétéroatomes tels que notamment l'oxygène, l'azote, le soufre et les halogènes.

Ces radicaux aliphatiques sont par exemple choisis parmi les radicaux alkyle, alcoxy, polyoxyalkylène en C₂-C₆, alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone.
- les sels d'ammonium quaternaire de l'imidazoline, comme par exemple ceux de formule (III) suivante : 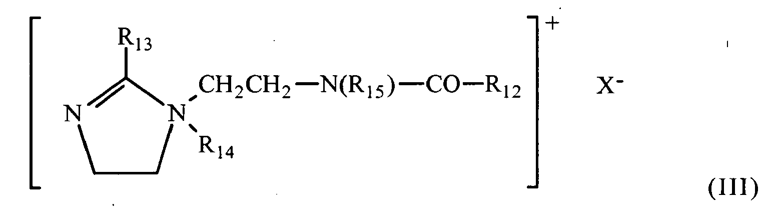 dans laquelle :
   R₁₂ représente un radical alcényle ou alkyle comportant de 8 à 30 atomes de carbone, par exemple dérivés des acides gras du suif,
   R₁₃ représente un atome d'hydrogène, un radical alkyle en C₁-C₄ ou un radical alcényle ou alkyle comportant de 8 à 30 atomes de carbone,
   R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates.

De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT® W 75 par la société REWO ;
- les sels de diammonium quaternaire de formule (IV) : 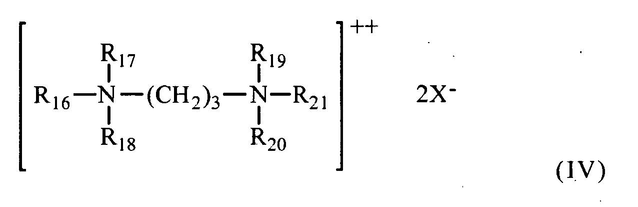 dans laquelle :
   R₁₆ désigne un radical aliphatique comportant environ de 16 à 30 atomes de carbone,
   R₁₇, R₁₈, R₁₉, R₂₀ et R₂₁, identiques ou différents sont choisis parmi l'hydrogène ou un radical alkyle comportant de 1 à 4 atomes de carbone, et
   X⁻ est un anion choisi dans le groupe des halogénures, acétates, phosphates, nitrates et méthylsulfates. De tels sels de diammonium quaternaire comprennent notamment le dichlorure de propane suif diammonium ;
- les sels d'ammonium quaternaire contenant au moins une fonction ester, tels que ceux de formule (V) suivante : 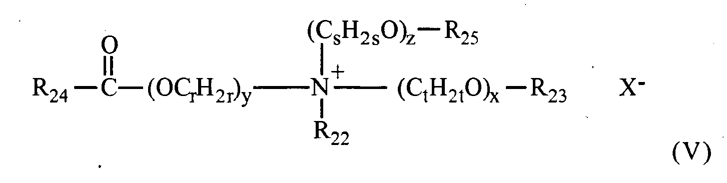 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi : 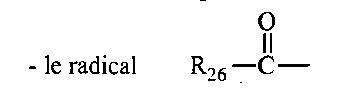
      - les radicaux R₂₇ hydrocarbonés en C₁-C₂₂, linéaires ou ramifiés, saturés ou insaturés,
      - l'atome d'hydrogène ;
   R₂₅ est choisi parmi :
      - le radical 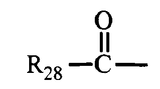
      - les radicaux R₂₉ hydrocarbonés en C₁-C₆, linéaires ou ramifiés, saturés ou insaturés,
      - l'atome d'hydrogène ;
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence, R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence de 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion X⁻ est de préférence un halogénure (chlorure, bromure ou iodure) ou un alkylsulfate, plus particulièrement un méthylsulfate. On peut cependant utiliser le méthanesulfonate, le phosphate, le nitrate, le tosylate, un anion dérivé d'acide organique tel que l'acétate ou le lactate, ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (V) dans laquelle :
R₂₂ désigne un radical méthyle ou éthyle,
x et y sont égaux à 1 ;
z est égal à 0 ou 1 ;
r, s et t sont égaux à 2 ;
R₂₃ est choisi parmi :
   - le radical 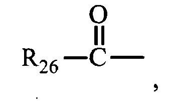
   - les radicaux méthyle, éthyle ou hydrocarbonés en C₁₄-C₂₂,
   - l'atome d'hydrogène;
R₂₅ est choisi parmi :
   - le radical 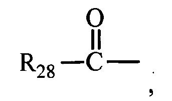
   - l'atome d'hydrogène ;
R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (V) tels que les sels (chlorure ou méthylsulfate notamment) de diacyloxyéthyl-diméthylammonium, de diacyloxyéthyl(hydroxyéthyl)méthylammonium, de monoacyloxyéthyl(dihydroxyéthyl)méthylammonium, de triacyloxyéthyl(méthyl)ammonium, de monoacyloxyéthyl-(hydroxyéthyl)diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl(dihydroxyéthyl)méthylammonium, 45 à 60 % de méthyl sulfate de diacyloxyéthyl(hydroxyéthyl)méthylammonium et 15 à 30 % de méthylsulfate de triacyloxyéthyl(méthyl)ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4,874,554 et US-A-4,137,180.

Parmi les sels d'ammonium quaternaire de formule (II), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl(myristylacétate)ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de cétyltriméthyl ammonium, le chlorure de béhényltriméthylammonium et le chlorure de palmitylamidopropyltriméthylammonium.

Les tensioactifs ioniques utilisables dans la composition cosmétique peuvent être également des tensioactifs anioniques.

Comme tensioactifs anioniques utilisables dans la composition cosmétique selon l'invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acyl sarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆-C₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyllactylates dont le groupe acyle comporte de 8 à 20 atomes de carbone.

En outre, on peut encore citer les acides alkyl-D-galactoside-uroniques et leurs sels ainsi que les acides alkyl(C₆-C₂₄)éthercarboxyliques polyoxyalkylénés, les acides alkyl(C₆-C₂₄)aryl(C₆-C₂₄) éthercarboxyliques polyoxyalkylénés, les acides alkyl(C₆-C₂₄) amidoéthercarboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

De préférence, le tensioactif ionique est un tensioactif cationique.

Les tensioactifs non ioniques utilisables dans la composition cosmétique de la présente invention sont des composés bien connus en soi (voir notamment à cet égard "Handbook of Surfactants" par M.R. PORTER, éditions Blackie & Son (Glasgow and London), 1991, p. 116-178). Ils sont choisis notamment parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les dérivés de N-(alkyle en C₆-C₂₄)glucamine, les oxydes d'amines tels que les oxydes d'(alkyle en C₁₀-C₁₄)amines ou les oxydes de N-(acyle en C₁₀-C₁₄)aminopropylmorpholine.

Parmi les tensioactifs non ioniques cités ci-dessus, on utilise de préférence les alcools polyéthoxylés, polypropoxylés ou polyglycérolés.

Le ou les tensioactifs sont présents à une concentration allant de 0,01 à 20 % en poids, de préférence à une concentration allant de 0,05 à 10 % en poids, et encore plus préférentiellement à une concentration allant de 0,1 à 5 % en poids, par rapport au poids total de la composition.

La composition cosmétique selon l'invention peut comprendre en outre un ou plusieurs adjuvants cosmétiques choisis parmi les agents conditionneurs de type esters, les agents anti-mousse, les agents hydratants, les agents émollients, les glycols, les plastifiants, les agents épaississants minéraux, les agents épaississants organiques, polymériques ou non, associatifs ou non, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents acidifiants, les agents anti-corrosion, les agents réducteurs ou anti-oxydants, les agents oxydants, les charges minérales, les paillettes.

L'homme du métier veillera à choisir les éventuels adjuvants et leur quantité de manière à ce qu'ils ne nuisent pas aux propriétés des compositions de la présente invention.

De préférence, le ou les adjuvants cosmétiques sont présents à une concentration allant de 0,001 à 50 % en poids, par rapport au poids total de la composition.

Par milieu cosmétiquement acceptable, on entend un milieu compatible avec les matières kératiniques et en particulier les cheveux.

Le milieu cosmétiquement acceptable peut être un milieu alcoolique, aqueux ou hydroalcoolique. Ainsi le milieu peut notamment être constitué uniquement par de l'eau ou d'un monoalcool ou par un mélange d'eau et d'un ou de plusieurs monoalcools cosmétiquement acceptables tels que les alcools inférieurs en C₁-C₄, les éthers de polyols présentant un hydroxyle libre et leurs mélanges. De préférence, l'alcool est l'éthanol.

La présente invention concerne aussi un procédé de traitement cosmétique des cheveux, par exemple de coiffage, qui consiste à appliquer une quantité efficace d'une composition décrite ci-dessus, sur les cheveux secs ou humides, à rincer ou non après un éventuel temps de pose ou après un éventuel séchage.

De préférence, la composition est non rincée.

La présente invention concerne également l'utilisation d'une composition cosmétique pour la fixation des fibres kératiniques, telles que les cheveux.

Les exemples suivants sont donnés à titre illustratif et non limitatif de la présente invention.

### EXEMPLES

Les compositions suivantes selon l'invention ont été préparées. Les données ci-dessous sont exprimées en % en poids de matière active, par rapport au poids total de la composition.

### Mousses coiffantes

| | 1 | 2 |
|---|---|---|
| Carraghénane Lambda ( Welgeenan ED 1039, vendu par Eurogum) | 1 % | 1,2 % |
| APG (Plantacare 2000 UP, vendu par Cognis) | 1,7 % | 1,3 % |
| Tensioactif ionique ou non ionique (Polysorbate-20) | 0,75 % | - |
| Polymère fixant non-ionique (PVP) | - | 3 % |
| Alcool dénaturé | - | 5 % |
| Gaz propulseur (hydrocarbures, CO₂, protoxyde d'azote...) | 3,5 % | 3,5 % |
| Conservateurs, neutralisant, parfum | q.s. | q.s. |
| Eau q.s.p. | 100 % | 100 % |

Cette composition incorporée dans un dispositif aérosol génère une mousse de qualité, qui confère aux cheveux mouillés un très bon effet traitant.

### Gels coiffants

| | 1 | 2 |
|---|---|---|
| Carraghénane Lambda (Satiagum UTC 10, vendu par Degussa) | 3,5 % | 4 % |
| APG (Oramix NS 10, vendu par Seppic) | 0,6 % | 0,8 % |
| Polymère fixant ionique ou non (PVP) | - | 2,5 % |
| Alcool dénaturé | - | 5 % |
| Conservateurs, neutralisant, parfum | q.s. | q.s. |
| Eau q.s.p | 100 % | 100 % |

Ce gel appliqué sur cheveux humides est peu collant. Le niveau de fixation est très satisfaisant.

### Conditionneurs

| | 1 | 2 |
|---|---|---|
| Carraghénane Lambda (Welgeenan ED 1039, vendu par Eurogum) | 3,2 % | 3 % |
| APG (Plantacare 2000 UP, vendu par Cognis) | 0,9 % | 1 % |
| Polymère fixant non-ionique (Copolymère 845) | - | 3 % |
| Alcool dénaturé | - | 5 % |
| Silicone (Diméthicone) | 2 % | - |
| Cations (Chlorure de cétrimonium) | - | 0,5 % |
| Conservateurs, neutralisant, parfum | q.s. | q.s. |
| Eau q.s.p | 100 % | 100 % |

Ce gel appliqué sur cheveux humides est peu collant et conduit à un brushing de qualité.

## Revendications

1. Composition cosmétique pour le traitement des fibres kératiniques, en particulier des fibres kératiniques humaines telles que les cheveux, **caractérisée par le fait qu'**elle comprend, dans un milieu cosmétiquement acceptable,
- au moins un polysaccharide de type carraghénane lambda, et
- au moins un agent tensioactif non-ionique de type alkylpolyglucoside, la teneur en polysaccharide de type carraghénane lambda étant comprise entre 0,1 et 30 % en poids du poids total de la composition, ***les valeurs aux bornes étant exclues**.*

2. Composition cosmétique selon la revendication 1, **caractérisée par le fait que** l'agent tensioactif non-ionique de type alkylpolyglucoside est un composé de formule (I) suivante :
R₁-O-(R₂-O)ₙ-(L)_{b} (I)
dans laquelle,
R₁ désigne un radical alkyle et/ou alcényle linéaire ou ramifié comportant de 8 à 24 atomes de carbone, un radical alkylphényle dont le groupe alkyle linéaire ou ramifié comporte de 8 à 24 atomes de carbone,
R₂ désigne un radical alkylène comportant de 2 à 4 atomes de carbone,
L désigne un sucre réduit comportant de 5 à 6 atomes de carbone,
a désigne une valeur allant de 0 à 10, et
b désigne une valeur allant de 1 à 15.

3. Composition cosmétique selon la revendication 2, **caractérisée par le fait que** l'agent tensioactif non-ionique de type alkylpolyglucoside est un composé de formule (I), dans laquelle
R₁ désigne un radical alkyle et/ou alcényle linéaire ou ramifié comportant de 9 à 14 atomes de carbone,
a désigne zéro,
L désigne le glucose,
b désigne une valeur allant de 1 à 4.

4. Composition cosmétique selon l'une quelconque des revendications 1 à 3, **caractérisée par le fait que** l'agent (ou les agents) tensioactif(s) non-ionique(s) de type alkylpolyglucoside peut (ou peuvent) être présent(s) dans la composition à une teneur comprise entre 0,05 et 30 % en poids du poids total de la composition cosmétique.

5. Composition cosmétique selon l'une quelconque des revendications 1 à 4, **caractérisée par le fait que** le poids moléculaire (PM) du polysaccharide de type carraghénane lambda est compris entre 100 000 et 1 000 000 u.m.a., de préférence entre 250 000 et 800 000 u.m.a.

6. Composition cosmétique selon l'une quelconque des revendications précédentes, **caractérisée par le fait que** le polysaccharide de type carraghénane lambda est présent dans la composition cosmétique en une quantité variant de 0,2 à 20 % en poids et plus préférentiellement en une quantité variant de 0,5 à 15 % en poids, par rapport au poids total de la composition.

7. Composition cosmétique selon l'une quelconque des revendications précédentes, **caractérisée par le fait qu'**elle comprend en outre au moins additif choisi parmi une silicone, un corps gras, un polymère fixant et un tensioactif.

8. Composition cosmétique selon l'une quelconque des revendications précédentes, **caractérisée par le fait qu'**elle comprend en outre au moins un adjuvant cosmétique choisi parmi les agents conditionneurs de type esters, les agents anti-mousse, les agents hydratants, les agents émollients, les glycols, les plastifiants, les agents épaississants minéraux, les agents épaississants organiques, polymériques ou non, associatifs ou non, les filtres solaires hydrosolubles et liposolubles, siliconés ou non siliconés, les colorants permanents ou temporaires, les parfums, les peptisants, les conservateurs, les céramides et pseudo-céramides, les vitamines et les provitamines dont le panthénol, les protéines, les agents séquestrants, les agents solubilisants, les agents alcanisants, les agents acidifiants, les agents anti-corrosion, les agents réducteurs ou anti-oxydants, les agents oxydants, les charges minérales, les paillettes.

9. Composition cosmétique selon l'une quelconque des revendications précédentes, **caractérisée par le fait que** le milieu cosmétiquement acceptable est un milieu aqueux, alcoolique ou hydroalcoolique.

10. Procédé de traitement cosmétique, **caractérisé par le fait qu'**il comprend l'application d'une composition cosmétique selon l'une quelconque des revendications 1 à 9, en particulier sur les fibres kératiniques, telles que les cheveux.

11. Procédé de traitement cosmétique selon la revendication 10, **caractérisé par le fait que** l'application de ladite composition n'est pas suivie par un rinçage.

12. Utilisation d'une composition cosmétique selon l'une quelconque des revendications 1 à 9 pour la fixation des fibres kératiniques, telles que les cheveux.

13. Utilisation d'une composition cosmétique selon l'une quelconque des revendications 1 à 9 pour le soin des fibres kératiniques, telles que les cheveux.

14. Utilisation pour la fixation des fibres kératiniques, telles que les cheveux, d'une composition cosmétique comprenant, dans un milieu cosmétiquement acceptable :
- au moins un polysaccharide de type carraghénane lambda, et
- au moins un agent tensioactif non-ionique de type alkylpolyglucoside.

15. Utilisation pour le soin des fibres kératiniques, telles que les cheveux, d'une composition cosmétique comprenant, dans un milieu cosmétiquement acceptable :
- au moins un polysaccharide de type carraghénane lambda, et
- au moins un agent tensioactif non-ionique de type alkylpolyglucoside.

## Claims

1. Cosmetic composition for the treatment of keratinous fibres, in particular human keratinous fibres, such as the hair, **characterized in that** it comprises, in a cosmetically acceptable medium,
- at least one lambda-carrageenan polysaccharide, and
- at least one alkyl polyglucoside non-ionic surfactant, the content of lambda-carrageenan polysaccharide being of between 0.1 and 30% by weight of the total weight of the composition, the values at the limits being excluded.

2. Cosmetic composition according to Claim 1, **characterized in that** the alkyl polyglucoside non-ionic surfactant is a compound of following formula (I):
R₁-O-(R₂-O)ₐ-(L)_{b} (I)
in which
R₁ represents a linear or branched alkyl and/or alkenyl radical comprising from 8 to 24 carbon atoms or an alkylphenyl radical, the linear or branched alkyl group of which comprises from 8 to 24 carbon atoms,
R₂ represents an alkylene radical comprising from 2 to 4 carbon atoms,
L represents a reduced sugar comprising from 5 to 6 carbon atoms,
a represents a value ranging from 0 to 10, and
b represents a value ranging from 1 to 15.

3. Cosmetic composition according to Claim 2, **characterized in that** the alkyl polyglucoside non-ionic surfactant is a compound of formula (I) in which
R₁ represents a linear or branched alkyl and/or alkenyl radical comprising from 9 to 14 carbon atoms,
a represents zero,
L represents glucose,
b represents a value ranging from 1 to 4.

4. Cosmetic composition according to any one of Claims 1 to 3, **characterized in that** the alkyl polyglucoside non-ionic surfactant(s) can be present in the composition in an amount ranging from 0.05 to 30% by weight of the total weight of the cosmetic composition.

5. Cosmetic composition according to any one of Claims 1 to 4, **characterized in that** the molecular weight (MW) of the lambda-carrageenan polysaccharide is of between 100 000 and 1 000 000 a.m.u., preferably between 250 000 and 800 000 a.m.u.

6. Cosmetic composition according to any one of the preceding claims, **characterized in that** the lambda-carrageenan polysaccharide is present in the cosmetic composition in an amount ranging from 0.2 to 20% by weight and more preferably in an amount ranging from 0.5 to 15% by weight, relative to the total weight of the composition.

7. Cosmetic composition according to any one of the preceding claims, **characterized in that** it additionally comprises at least one additive chosen from a silicone, a fatty substance, a fixing polymer and a surfactant.

8. Cosmetic composition according to any one of the preceding claims, **characterized in that** it additionally comprises at least one cosmetic adjuvant chosen from conditioning agents of ester type, antifoaming agents, moisturizing agents, emollients, glycols, plasticizers, inorganic thickening agents, polymeric or non-polymeric and associative or non-associative organic thickening agents, silicone or non-silicone and water-soluble or fat-soluble sunscreens, permanent or temporary colourings, fragrances, peptizing agents, preservatives, ceramides and pseudoceramides, vitamins and provitamins, including panthenol, proteins, sequestering agents, solubilizing agents, basifying agents, acidifying agents, corrosion inhibitors, reducing agents or antioxidants, oxidizing agents, inorganic fillers or glitter.

9. Cosmetic composition according to any one of the preceding claims, **characterized in that** the cosmetically acceptable medium is an aqueous, alcoholic or aqueous/alcoholic medium.

10. Cosmetic treatment method, **characterized in that** it comprises the application of a cosmetic composition according to any one of Claims 1 to 9 in particular to keratinous fibres, such as the hair.

11. Cosmetic treatment method according to Claim 10, **characterized in that** the application of the said composition is not followed by a rinsing.

12. Use of a cosmetic composition according to any one of Claims 1 to 9 for fixing keratinous fibres, such as the hair.

13. Use of a cosmetic composition according to any one of Claims 1 to 9 for caring for keratinous fibres, such as the hair.

14. Use for the fixing of keratinous fibres, such as the hair, of a cosmetic composition comprising, in a cosmetically acceptable medium:
- at least one polysaccharide of lambda-carrageenan type, and
- at least one non-ionic surface-active agent of alkylpolyglucoside type.

15. Use for caring for keratinous fibres, such as the hair, of a cosmetic composition comprising, in a cosmetically acceptable medium:
- at least one lambda-carrageenan polysaccharide, and
- at least one alkyl polyglucoside non-ionic surfactant.

## Patentansprüche

1. Kosmetische Zusammensetzung zur Behandlung von Keratinfasern und insbesondere menschlichen Keratinfasern, wie Haaren, **dadurch gekennzeichnet, dass** sie in einem kosmetisch akzeptablen Medium enthält:
■ mindestens ein Lambdacarrageenan Polysaccharid und
■ mindestens einen Alkylpolyglucosid nichtionischen grenzflächenaktiven Stoff, wobei der Mengenteil des Lambdacarrageenan Polysaccharids im Bereich von 0,1 bis 30 Gew.-% des Gesamtgewichts der Zusammensetzung liegt, wobei die Grenzwerte ausgenommen sind.

2. Kosmetische Zusammensetzung nach Anspruch 1, **dadurch gekennzeichnet, dass** der Alkylpolyglucosid nichtionische grenzflächenaktive Stoff eine Verbindung der folgenden Formel (I)
R₁-O-(R₂-O)ₐ-(L)_{b} (I)
wobei
R₁ steht eine geradkettige oder verzweigte Alkyl- und/oder Alkenylgruppe, die 8 bis 24 Kohlenstoffatome enthält, oder eine Alkylphenylgruppe, deren geradkettige oder verzweigte Alkylgruppe 8 bis 24 Kohlenstoffatome enthält,
R₂ steht eine Alkylengruppe, die von 2 bis 4 Kohlenstoffatome enthält,
L ist ein reduzierter Zucker mit 5 bis 6 Kohlenstoffatomen,
a steht einen Wert im Bereich von 0 bis 10, und
b steht einen Wert im Bereich von 1 bis 15.

3. Kosmetische Zusammensetzung nach Anspruch 2, **dadurch gekennzeichnet, dass** der Alkylpolyglucosid nichtionische grenzflächenaktive Stoff eine Verbindung der Formel (I) ist, worin R₁ eine geradkettige oder verzweigte Alkyl- und/oder Alkenylgruppe steht, die 9 bis 14 Kohlenstoffatome enthält,
a Null ist,
L Glucose ist,
b einen Wert im Bereich von 1 bis 4 steht.

4. Kosmetische Zusammensetzung nach einem der Ansprüche 1 bis 3, **dadurch gekennzeichnet, dass** der (oder die) Alkylpolyglucosid nichtionische(n) grenzflächenaktive(n) Stoff(e) in der Zusammensetzung in einem Mengenanteil im Bereich von 0,05 bis 30 Gew.-% des Gesamtgewichts der kosmetische Zusammensetzung enthalten sein kann (oder können).

5. Kosmetische Zusammensetzung nach einem der Ansprüche 1 bis 4, **dadurch gekennzeichnet, dass** das Molekulargewicht (MG) des Lambdacarrageenan Polysaccharids im Bereich von 100.000 bis 1.000.000 u und vorzugsweise 250.000 bis 800.000 u liegt.

6. Kosmetische Zusammensetzung nach einem der vorhergehenden Ansprüche, **dadurch gekennzeichnet, dass** das Lambdacarrageenan Polysaccharid in der kosmetischen Zusammensetzung in einem Mengenanteil im Bereich von 0,2 bis 20 Gew.-% und vorzugsweise in einem Mengenanteil im Bereich von 0,5 bis 15 Gew.-%, bezogen auf das Gesamtgewicht der Zusammensetzung, enthalten ist.

7. Kosmetische Zusammensetzung nach einem der vorhergehenden Ansprüche, **dadurch gekennzeichnet, dass** sie ferner mindestens einen Zusatzstoff enthält, der unter den Siliconen, Fettsubstanzen, fixierenden Polymeren und grenzflächenaktiven Stoffen ausgewählt ist.

8. Kosmetische Zusammensetzung nach einem der vorhergehenden Ansprüche, **dadurch gekennzeichnet, dass** sie ferner mindestens einen kosmetischen Zusatzstoff enthält, der ausgewählt ist unter: Ester Konditioniermitteln, Schaumverhütungsmitteln, Hydratisierungsmitteln, Emollientien, Glycolen, Weichmachern, anorganischen Verdickungsmittel, organischen Verdickungsmittel, die polymer oder nicht polymer, assoziativ oder nicht assoziativ sind, wasserlöslichen und fettlöslichen Sonnenschutzfiltern, die siliconiert oder nicht siliconiert sind, dauerhaften oder temporären Farbmitteln, Parfums, peptisierenden Stoffen, Konservierungsmitteln, Ceramiden und Pseudoceramiden, Vitaminen und Provitaminen, darunter Panthenol, Proteinen, Maskierungsmitteln, Lösungsmitteln, Alkalisierungsmitteln, Ansäuerungsmitteln, Korrosionsschutzmitteln, Reduktionsmitteln oder Antioxidantien, Oxidationsmitteln, anorganischen Füllstoffen und Pailletten.

9. Kosmetische Zusammensetzung nach einem der vorhergehenden Ansprüche, **dadurch gekennzeichnet, dass** das kosmetisch akzeptable Medium ein wässriges, alkoholisches oder wässrigalkoholisches Medium ist.

10. Kosmetisches Verfahren zur Behandlung, **dadurch gekennzeichnet, dass** es die Anwendung einer kosmetischen Zusammensetzung nach einem der Ansprüche 1 bis 9 insbesondere auf Keratinfasern wie Haare umfasst.

11. Kosmetisches Verfahren zur Behandlung nach Anspruch 10, **dadurch gekennzeichnet, dass** nach der Anwendung der Zusammensetzung kein Spülschritt folgt.

12. Verwendung einer kosmetischen Zusammensetzung nach einem der Ansprüche 1 bis 9 für die Festigung von Keratinfasern wie Haaren.

13. Verwendung einer kosmetischen Zusammensetzung nach einem der Ansprüche 1 bis 9 für die Pflege von Keratinfasern wie Haaren.

14. Verwendung einer kosmetischen Zusammensetzung, die in einem kosmetisch akzeptablen Medium enthält:
- mindestens ein Polysaccharid vom Lambdacarrageenan Polysaccharid und
- mindestens einen nichtionischen grenzflächenaktiven Stoff vom Alkylpolyglucosid-Typ,
für die Festigung von Keratinfasern wie Haaren.

15. Verwendung einer kosmetischen Zusammensetzung, die in einem kosmetisch akzeptablen Medium enthält:
- mindestens ein Polysaccharid vom lambda-Carrageen-Typ und
- mindestens einen Alkylpolyglucosid nichtionischen grenzflächenaktiven Stoff,
für die Pflege von Keratinfasern wie Haare.
